(19) Europäisches Patentamt
European Patent Office
Office européen des brevets

(11) Veröffentlichungsnummer: **0 252 353**
**B1**

(12) # EUROPÄISCHE PATENTSCHRIFT

(45) Veröffentlichungstag der Patentschrift: **29.08.90**

(51) Int. Cl.[5]: **C07D 207/38**

(21) Anmeldenummer: **87108902.5**

(22) Anmeldetag: **22.06.87**

(54) **4-Benzyloxy-3-pyrrolin-2-on-1-yl-acetamid, dessen Herstellung und Verwendung.**

(30) Priorität: **26.06.86 CH 2567/86**

(43) Veröffentlichungstag der Anmeldung: **13.01.88 Patentblatt 88/2**

(45) Bekanntmachung des Hinweises auf die Patenterteilung: **29.08.90 Patentblatt 90/35**

(84) Benannte Vertragsstaaten: **AT BE CH DE ES FR GB IT LI LU NL SE**

(56) Entgegenhaltungen:
**DE-A-2 328 277**
**US-A-3 299 095**

**ILL FARMACO ED. SC., Band 32, Nr. 8, 1977, Seiten 602-613; G. PIFFERI et al.: "Cyclic gaba-gabob analogues"**

(73) Patentinhaber: **LONZA AG, Gampel/Wallis(CH)**

(72) Erfinder: **Meul, Thomas, Dr., Balfrinstrasse 21, VISP Kanton Wallis(CH)**

(74) Vertreter: **Weinhold, Peter, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40(DE)**

**EP 0 252 353 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Das neue 4-Benzyloxy-3-pyrrolin-2-on-1-yl-acetamid ist ein wertvolles Zwischenprodukt für die Synthese des cerebral wirksamen 4-Hydroxy-pyrrolidin-2-on-1-yl-acetamids (Oxiracetam).

Von Pifferi et al, Il Farmaco, Ed. Sc., 1977, 32, 602 ist ein Verfahren bekannt, den Wirkstoff herzustellen.

Eine schlechte Ausbeute und teure Ausgangsprodukte machen dieses Verfahren jedoch nicht rentabel.

Es stellte sich daher die Aufgabe, einen Weg zu finden, der ohne die genannten Nachteile auskommt.

Diese Aufgabe konnte bemerkenswert einfach gelöst werden durch das Auffinden der neuen Zwischenstufe 4-Benzyloxy-3-pyrrolin-2-on-1-yl-acetamid.

Zugänglich ist diese Zwischenstufe gemäss Patentanspruch 2, entweder aus einem 4-($C_1$-$C_2$)-alkoxy-3-pyrrolin-2-on oder einem 4-($C_1$-$C_2$)-alkoxy-3-pyrrolin-2-on-1-yl-essigsäure-($C_1$-$C_4$)-alkylester.

Die letzteren sind auf einfache Weise nach der EP-A 0 216 326 herstellbar.

Es sind ohne weiteres Ausgangsprodukte mit längeren Alkoxyresp. Alkylgruppen einsetzbar. Da diese Gruppen im Laufe des Verfahrens wieder abgespalten werden,sind solche Verbindungen aber nicht interessant.

Vom 4-($C_1$-$C_2$)-alkoxy-3-pyrrolin-2-on ausgehend wird in einer ersten Stufe in Gegenwart einer Säure mit Benzylalkohol zum 4-Benzyloxy-3-pyrrolin-2-on umgesetzt.

Als Säuren werden für diesen Schritt zweckmässig Sulfonsäuren, wie Methansulfonsäure oder p-Toluolsulfonsäure in katalytischen Mengen von zweckmässig 0,05 bis 0,2 Mol eingesetzt.

Es ist von Vorteil, direkt im Benzylalkohol als Lösungsmittel zu arbeiten.

Der Benzylalkohol wird zweckmässig in einer Menge von 1,5 bis 5 Mol pro Mol Ausgangsprodukt eingesetzt.

Die Reaktionstemperatur liegt vorteilhaft in einem Bereich von 60 bis 100°C.

Da es sich bei dieser Reaktion formal um eine Umesterungsreaktion handelt, ist es von Vorteil, die Reaktion bei vermindertem Druck vorzugsweise zwischen 10 bis 50 mbar durchzuführen, um so die abgespaltenen, niedrig siedenden Alkohole aus dem Gleichgewicht zu entfernen.

Nach beendeter Reaktion, d.h., nach ungefähr 10 bis 25 Stunden (je nach Katalysator-Menge Sulfonsäure) kann das 4-Benzyloxy-3-pyrrolin-2-on auf übliche Weise durch z.B. azeotropes Abtrennen des überschüssigen Benzylalkohols und gegebenenfalls durch Kristallisation des erhaltenen Produktes aufgearbeitet werden.

In einer zweiten Stufe wird das erhaltene Produkt mit einem Bromessigsäure-($C_1$-$C_4$)-alkylester in Gegenwart eines Alkali hydrids zum 4-Benzyloxy-3-pyrrolin-2-on-1-yl-essigsäure-($C_1$-$C_4$)-alkylester umgesetzt.

Als bevorzugter Bromessigsäurealkylester gelangt der Bromessigsäureethylester in einer Menge von zweckmässig 1 bis 1,5 Mol pro Mol 4-Benzyloxy-3-pyrrolin-2-on zur Anwendung.

Als bevorzugtes Alkalihydrid wird Natriumhydrid in einer Menge von 1 bis 1,5 Mol pro Mol 4-Benzyloxy-3-pyrrolin-2-on eingesetzt.

Vorteilhaft wird in polaren aprotischen Lösungsmitteln wie Dimethylformamid, Dimethylacetamid, Acetonitril, besonders vorteilhaft in Acetonitril als Lösungsmittel, bei Reaktionstemperaturen von 0 bis 40°C gearbeitet.

Der resultierende 4-Benzyloxy-3-pyrrolin-2-on-1-yl-essigsäur-($C_1$-$C_4$)-alkylester kann dann in einer letzten Stufe mit Ammo niak zum 4-Benzyloxy-3-pyrrolin-2-on-1-yl-acetamid umgesetzt werden.

Zweckmässig wird dabei so vorgegangen, dass das Ausgangsprodukt vorteilhaft in einem Alkohol wie Methanol oder Ethanol gelöst wird, wobei der Alkohol vorher mit gasförmigem Ammoniak bei -10 bis 0°C gesattigt wird und darauf im Autoklaven die Reaktionsmischung bei 60 bis 80°C, 10 bis 15 Stunden gerührt wird.

Nach üblicher Aufarbeitung kann das gewünschte Zwischenprodukt nach Patentanspruch 1 in guter Ausbeute und Qualität erhalten werden.

Wird vom 4-($C_1$-$C_2$)-Alkoxy-3-pyrrolin-2-on-1-yl-essigsäure-($C_1$-$C_4$)-alkylester ausgegangen, wird in einem ersten Schritt mit Benzylalkohol, sauer zum 4-Benzyloxy-3-pyrrolin-2-on-1-yl-essigsäurebenzylester umgesetzt.

Als Säuren werden für diesen Schritt zweckmässig Sulfonsäuren, vorzugsweise Methansulfonsäure oder p-Toluolsulfonsäure in katalytischen Mengen von zweckmässig 0,05 bis 0,2 Mol eingesetzt. Es ist von Vorteil, direkt im Benzylalkohol als Lösungsmittel zu arbeiten.

Der Benzylalkohol wird zweckmässig in einer Menge von 2,5 bis 5,0 Mol pro Mol Ausgangsprodukt eingesetzt.

Die Reaktionstemperatur liegt vorteilhaft in einem Bereich von 80 bis 120°C.

Wie bei der Herstellung des 4-Benzyloxy-3-pyrrolin-2-ons, ist es von Vorteil, die Reaktion bei vermindertem Druck, vorzugsweise zwischen 10 bis 50 mbar durchzuführen.

Nach beendeter Reaktion, d.h., nach ca. 7 bis 10 Stunden kann der 4-Benzyloxy-3-pyrrolin-2-on-1-yl-essigsäurebenzylester nach üblicher Aufarbeitung und gegebenenfalls durch Reinigung mittels Umkristallisation erhalten werden.

In einer letzten Stufe kann dann mit Ammoniak zum gewünschten Zwischenprodukt zum 4-Benzyloxy-3-pyrrolin-2-on-1-yl-acetamid umgesetzt werden.

Vorteilhaft wird dazu der als Lösungsmittel fungierende Alkohol, bevorzugt Methanol und Ethanol, bei -10 bis 0°C mit gasförmigem Ammoniak gesättigt, dann der 4-Benzyloxy-3-pyrrolin- 2-on-1-yl-essigsäurebenzylester zugegeben und die Reaktionsmischung schliesslich im Autoklaven bei 60 bis 80°C, 6 bis 10 Stunden gerührt.

Nach einfacher Aufarbeitung kann das gewünschte Zwischenprodukt in guter Ausbeute und Qualität erhalten werden.

Gemäss Patentanspruch 7 kann das 4-Benzyloxy-3-pyrrolin-2-on-1-yl-acetamid als besonders

vorteilhaftes Zwischenprodukt zur Herstellung des cerebral wirksamen 4-Hydroxy-pyrrolidin-2-on-1-yl-acetamids eingesetzt werden.
Zweckmässig wird dazu in einem ersten Schritt katalytisch hydrogenolysiert.
Als Katalysatoren gelangen zweckmässig Edelmetallkatalysatoren zur Anwendung, die eine selektive Debenzylierung bewirken. Vorzugsweise wird Palladium, aufgebracht auf einem üblichen Trägermaterial, bevorzugt auf Aktivkohle mit einem Katalysatorgehalt von 1 bis 10 % angewendet.
Die Katalysatormenge bewegt sich zweckmässig zwischen 5 und 10 Gew%, bezogen auf die Menge eingesetztes 4-Benzyloxy-3-pyrrolin-2-on-1-yl-acetamid.

Wird das intermediär entstehende 2,4-Dioxopyrrolidin-1-yl acetamid mit komplexen Borhydriden, vorzugsweise mit Alkaliborhydriden, besonders bevorzugt mit Natriumborhydrid zum Endprodukt reduziert. So ist es von Vorteil, die Debenzylierung in polaren aprotischen Lösungsmitteln, wie Dimethylformamid oder Dimethylacetamid, unter einem Druck von zweckmässig 1 bis 20 bar und bei Temperaturen zwischen zweckmässig 0 bis 30°C durchzuführen.
Das Alkaliborhydrid wird dann vorzugsweise in einer Menge von 0,5 bis 0,8 Mol pro Mol 4-Benzyloxy-3-pyrrolin-2-on-1-yl-acetamid eingesetzt.
Unter Beibehaltung des polaren aprotischen Lösungsmittels arbeitet man zweckmässig bei Temperaturen von 0 bis 30°C. Auf diese Weise kommt ein entsprechender Vorteil des hier beschriebenen Verfahrens zum Tragen, denn das empfindliche, zur Dimerisierung neigende 2,4-Dioxo-pyrrolidin-1-yl-acetamid wird in Lösung gehalten und muss nicht isoliert werden, was jedoch möglich ist.

Wählt man die katalytische Hydrierung für die Umsetzung des intermediären 2,4-Dioxo-pyrrolidin-1-yl-acetamids zum Endprodukt, so arbeitet man für die katalytische Hydrogenolyse vorteilhaft in polaren protischen wasserfreien Lösungsmitteln, wie Methanol, Ethanol oder Essigsäure, bei zweckmässig 1 bis 20 bar und Temperaturen von zweckmässig 0 bis 30°C. Katalysator und Katalysatormenge bleiben gegenüber der katalytischen Hydrogenolyse in polaren aprotischen Lösungsmitteln vorteilhaft unverändert.
Die katalytische Hydrierung wird dann zweckmässig mit Platin-Katalysatoren, wie Platinoxid oder Platin, aufgebracht in einer Menge von 1 bis 10 % auf einen Träger, bevorzugt auf Aktivkohle, durchgeführt.
Die Katalysatormenge bewegt sich zweckmässig zwischen 1 und 10 Gew. % bezogen auf 4-Benzyloxy-3-pyrrolin-2-on-1-yl-acet amid. Der Wasserstoffdruck liegt zweckmässig zwischen 5 und 20 bar, die Temperatur zwischen 0 und 30°C.
Auch bei dieser katalytischen Reduktion ist es von Vorteil, das intermediär gebildete 2,4-Dioxo-pyrrolidin-1-yl-acetamid in Lösung zu behalten und nicht zu isolieren.

Besonders vorteilhaft ist es, die Debenzylierung des 4-Benzyloxy-3-pyrrolin-2-on-1-yl-acetamids durch katalytische Hydrogenolyse und die katalytische Reduktion durch Hydrierung des intermediären 2,4-Dioxo-pyrrolidin-1-yl-acetamids mit Wasserstoff als Eintopfverfahren mit Palladium/Platin-Mischkatalysatoren durchzuführen.
Wiederum werden zweckmässig polare protische wasserfreie Lösungsmittel, wie Methanol, Ethanol oder wasserfreie Essigsäure verwendet.

Der Wasserstoffdruck bewegt sich vorteilhaft zwischen 5 und 20 bar, die Temperaturen zwischen 0 und 30°C.

Zweckmässig wird ein Palladium/Platin-Mischkatalysator verwendet, dessen Verhältnis Palladium zu Platin 5 zu 1 bis 1 zu 2 beträgt.
Der Palladiumanteil liegt bevorzugt in einer Menge von 1 bis 10 %, aufgebracht auf einen üblichen Träger, bevorzugt auf Kohle, vor.
Der Platinanteil kann als Platinoxid oder ebenfalls als Platin, aufgebracht in einer Menge von 1 bis 10 %, auf einem üblichen Träger, bevorzugt auf Kohle, vorliegen.

Der Mischkatalysator wird in einer Menge von zweckmässig 5 bis 15 Gew.%, bezogen auf 4-Benzyloxy-3-pyrrolin-2-on-1-yl-acetamid eingesetzt.

Die Aufarbeitung des Endprodukts kann, unabhängig davon welches Verfahren gewählt wurde, auf verfahrensübliche Art und Weise erfolgen.

Das resultierende 4-Hydroxy-pyrrolidin-2-on-1-yl-acetamid kann gegebenenfalls durch eine Umkristallisation gereinigt werden.

Ueber das neue 4-Benzyloxy-3-pyrrolin-2-on-1-yl-acetamid als neues Zwischenprodukt und mit Hilfe der genannten erfindungsgemässen Verfahren gelingt es, auf beschriebene Weise das 4-Hydroxy-pyrrolidin-2-on-1-yl-acetamid in guten Ausbeuten in Reinheiten von grösser als 98 % herzustellen.

Beispiel 1

a) Herstellung von 4-Benzyloxy-3-pyrrolin-2-on-1-yl-essigsäure-benzylester

20,8 g 4-Methoxy-3-pyrrolin-2-on-1-yl-essigsäureethylester wurden in 44,1 g Benzylalkohol gelöst, mit 1,0 g Methansulfonsäure versetzt und bei 80°C und 20 mbar 12 Stunden gerührt. Anschliessend wurde die Reaktionslösung mit 250 ml Methylenchlorid und 500 ml Eiswasser versetzt und mit 10 ml gesättigter $NaHCO_3$-Lösung neutralisiert. Nach Trocknen der organischen Phase über $Na_2SO_4$ und Eindampfen des Lösungsmittels wurde der Rückstand mit 400 ml einer 2:1-Mischung aus Eiswasser und Ethanol versetzt. Dabei kristallisierte das Produkt aus. Die Ausbeute betrug 25,8 g (76 %). Das DC zeigte keine Nebenprodukte. Schmelzpunkt 92 bis 94°C.
NMR: (300 MHz, $CDCl_3$) $\delta$ in ppm
7,45-7,29 (m, 10 H), 5,20 (s, 1H), 5,16 (s, 2H), 4,98 (s, 2H), 4,22 (s, 2H), 4,03 (s, 2H).
MS (70 eV): 337 ($M^+$, 2), 246 (7), 202 (14), 145 (10), 91 (100), 65 (12).

b) Herstellung von 4-Benzyloxy-3-pyrrolin-2-on-1-yl-acetamid

25,0 g 4-Benzyloxy-3-pyrrolin-2-on-1-yl-essigsäurebenzylester wurden in 500 ml Methanol gelöst und bei 40°C unter Einleitung von gasförmigem $NH_3$

5 Stunden gerührt. Anschliessend wurde die Reaktionslösung eingedampft und der Rückstand mit 50 ml Aceton versetzt. Die ausgefallenen Kristalle wurden agenutscht und getrocknet.
Man erhielt 15,2 g DC-reines Produkt mit einem Schmelzpunkt von 174,5 bis 175,5°C.
NMR: (300 MHz, DMSO-$d_6$) δ in ppm
7,50-7,32 (m, 6H), 7,05 (br. s, 1H), 5,27 (s, 1H), 5,06 (s, 2H), 4,02 (s, 2H), 3,85 (s, 2H).
MS (70 eV): 246 ($M^+$, 20), 229 (23), 202 (49), 145 (30), 91 (100), 65 (52).

Beispiel 2

a) Direkte Herstellung von 4-Benzyloxy-3-pyrrolin-2-on-1-yl-acetamid aus 4-Methoxy-3-pyrrolin-2-on-1-yl-essigsäuremethylester

25,0 g (0,132 Mol) 4-Methoxy-3-pyrrolin-2-on-1-yl-essigsäuremethylester (98,0 %-ig), 41,7 g (0,38 Mol) Benzylalkohol und 1,9 g (19,8 mMol) Methansulfonsäure wurden während 8 Stunden bei 110°C im Wasserstrahlvakuum bei 20 mbar gerührt. Anschliessend wurde mit 167 ml Methylenchlorid verdünnt und mit 84 ml Eiswasser versetzt. Mit 19,8 ml gesättigter $NaHCO_3$-Lösung wurde die wässrige Phase neutralisiert und zweimal mit je 70 ml Methylenchlorid extrahiert.
Die organischen Phasen wurden vereinigt, über $Na_2SO_4$ getrocknet und am Rotationsverdampfer eingeengt. Der Rückstand (57,7 g) wurde zu einer bei - 10°C mit gasförmigem $NH_3$ gesättigten Methanol-Lösung (34 ml) gegeben und im Autoklav bei 70 bis 80°C 9 Stunden gerührt. Anschliessend wurde das Methanol abgedampft, der Rückstand mit 100 ml Tetrachlorkohlenstoff versetzt, auf 5°C gekühlt und die ausgefallenen Kristalle abgenutscht.
Das Rohprodukt (32,0 g) wurde aus 31 ml Wasser heiss umkristallisiert. Man erhielt 26,3 g nahezu weisses Produkt mit einem Gehalt nach HPLC von 97,9 %.
Ausbeute: 79,4 %.

Beispiel 3

a) Herstellung von 4-Benzyloxy-3-pyrrolin-2-on

5,7 g 4-Methoxy-3-pyrrolin-2-on und 10,8 g Benzylalkohol wurden mit 0,4 g Methansulfonsäure versetzt und 24 Stunden bei 80°C und 20 mbar gerührt. Anschliessend wurde die Reaktionslösung mit 50 ml Eiswasser und 100 ml Methylenchlorid versetzt und mit 4 ml gesättigter $NaHCO_3$-Lösung neutralisiert. Die wässrige Phase wurde noch zweimal mit je 50 ml Methylenchlorid extrahiert. Nach Trocknen der organischen Phase über $Na_2SO_4$ und Abdestillieren des Lösungsmittels wurde der Rückstand mit 150 ml Eiswasser versetzt, auf 100°C erhitzt und 100 ml Wasser-Benzylalkohol azeotrop abdestilliert. Die beim Abkühlen ausgefallenen Kristalle wurden aus 50 ml Toluol heiss umkristallisiert.
Man erhielt 6,7 g weisses, kristallines Produkt mit einem Schmelzpunkt von 147 bis 148°C.
NMR: (300 MHz, DMSO-$d_6$)
7,38 (m, 5H), 6,20 (br. s, 1H), 5,16 (s, 1H), 4,98 (s, 2H), 3,98 (s, 2H).
MS: (70 eV)
189($M^+$, 40), 172 (18), 132 (51), 91 (100).

b) Herstellung von 4-Benzyloxy-3-pyrrolin-2-on-1-yl-acetamid aus 4-Benzyloxy-3-pyrrolin-2-on

8,4 g 4-Benzyloxy-3-pyrrolin-2-on und 9,8 g Bromessigsäureethylester (95 %-ig) wurden in 50 ml wasserfreiem Acetonitril gelöst und auf 0°C gekühlt. Zu dieser Reaktionslösung gab man innerhalb von 20 Minuten 1,67 g Natriumhydrid (80 %-ig in Weissöl). Man liess 2 Stunden nachrühren, säuerte mit konz. Salzsäure auf pH 6 bis 7 an und destillierte das Lösungsmittel ab. Der Rückstand wurde in Wasser/Methylenchlorid aufgenommen. Nach Abtrennen der organischen Phase erhielt man 14,4 g Rohprodukt. Dieses Rohprodukt wurde in 20 ml Methanol, das vorher bei -10 bis 0°C mit gasförmigem Ammoniak gesättigt wurde, gelöst. Im Autoklav wurde die Reaktionslösung 12 Stunden bei 60 bis 80°C gerührt. Nach Abdestillieren des Methanols und Waschen des Rohproduktes mit Tetrachlorkohlenstoff wurde aus Wasser umkristallisiert.
Man erhielt 5,1 g DC-reines Produkt.

Beispiel 4

Katalytische Hydrogenolyse und Hydrierung des 4-Benzyloxy-3-pyrrolin-2-on-1-yl-acetamids

3,00 g 4-Benzyloxy-3-pyrrolin-2-on-1-yl-acetamid wurden in 30 ml konz. Essigsäure gelöst und mit 240 mg Palladium 5 % auf Kohle und 24 mg Platinoxid versetzt. Man hydrogenolysierte und hydrierte bei 15 bar Wasserstoffdruck und Raumtemperatur über 65 Stunden. Anschliessend wurde vom Katalysator abfiltriert und das Lösungsmittel abgedampft. Der Rückstand wurde in $H_2O$ aufgenommen und über 5,0 g schwach basischen Ionenaustauscher laufengelassen. Man destillierte das Wasser ab und versetzte den Rückstand mit 10 ml Aceton.
Man erhielt 1,53 g nahezu weisses 4-Hydroxy-pyrrolidin-2-on-1-yl-acetamid mit einem Schmelzpunkt von 163,5 bis 165,7°C.

Beispiel 5

Hydrogenolyse und $NaBH_4$-Reduktion des 4-Benzyloxy-3-pyrrolin-2-on-1-yl-acetamids

10,0 g 4-Benzyloxy-3-pyrrolin-2-on-1-yl-acetamid wurden in 33 ml Dimethylformamid, in Gegenwart von 800 mg Palladium 5 % auf Kohle bei einem $H_2$-Druck von 20 bar und bei Raumtemperatur über 5 Stunden hydrogenolysiert. Anschliessend filtrierte man den Katalysator ab und tropfte das Filtrat innerhalb einer Stunde bei Raumtemperatur zu einer Lösung von 1,1 g Natriumbor hydrid in 17 ml Dimethylformamid. Man liess 2 Stunden nachrühren und säuerte die Reaktionslösung mit 3 ml einer 1 zu 1 Mischung aus Ameisensäure und Methanol an. Die Lösungsmittel wurden abdestilliert. Der Rückstand

wurde in 100 ml Eiswasser aufgenommen, darauf über 80 g stark sauren und anschliessend über 80 g schwach basischen Ionenaustauscher filtriert. Die wässrige Lösung wurde eingedampft und der Rückstand in 70 ml Methanol aufgenommen.
Man destillierte 50 ml Methanol ab, wobei das 4-Hydroxy-pyrrolidin-2-on-1-yl-acetamid als mikrokristalliner Niederschlag ausfiel.
Man erhielt 4,2 g Produkt mit einem Schmelzpunkt von 166,2 bis 167,3°C (HPLC-Gehalt 96,9 %).
Umkristallisation aus Essigsäure/Aceton im Verhältnis 1 zu 3 lieferte ein Produkt mit einem Schmelzpunkt von 168 bis 169,5°C und einem HPLC-Gehalt von 99,0 %.

**Patentansprüche für die Vertragsstaaten: AT, BE, CH, DE, FR, GB, IT, LI, LU, NL, SE**

1. 4-Benzyloxy-3-pyrrolin-2-on-1-yl-acetamid der Formel

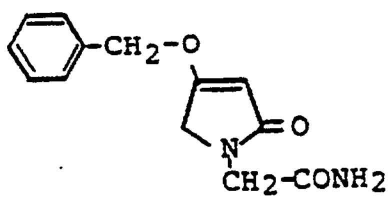

2. Verfahren zur Herstellung der Verbindung gemäss Patentanspruch 1, dadurch gekennzeichnet, dass man entweder ein 4-($C_1$-$C_2$)-Alkoxy-3-pyrrolin-2-on mit Benzylalkohol in Gegenwart einer Säure zum 4-Benzyloxy-3-pyrrolin-2-on umestert, mit 2-Bromessigsäure ($C_1$-$C_4$)-alkylester in Gegenwart eines Alkalihydrids zum 4-Benzyloxy-3-pyrrolin-2-on-1-yl-essigsäure-($C_1$-$C_4$)-alkylester alkyliert und schliesslich mit Ammoniak zum Endprodukt umsetzt, oder einen 4-($C_1$-$C_2$)-Alkoxy-3-pyrrolin-2-on-1-yl-essigsäure-($C_1$-$C_4$)-alkylester mit Benzylalkohol in Gegenwart einer Säure zum 4-Benzyloxy-3-pyrrolin-2-on-1-yl-essigsäurebenzylester und weiter - gegebenenfalls ohne dessen Isolierung mit Ammoniak zum Endprodukt umsetzt.

3. Verfahren nach Patentanspruch 2, dadurch gekennzeichnet, dass als Säure für die Umesterung des 4-($C_1$-$C_2$)-alkoxy-3-pyrrolin-2-ons oder des 4-($C_1$-$C_2$)-a1koxy-3-pyrrolin-2-on-1-yl-essigsäure-($C_1$-$C_4$)-alkylesters Sulfonsäuren eingesetzt werden

4. Verfahren nach Patentansprüchen 2 und 3, dadurch gekennzeichnet, dass die Umesterung des 4-($C_1$-$C_2$)-alkoxy-3-pyrrolin-2-ons bei Temperaturen von 60 bis 100°C bei vermindertem Druck bzw. die Umesterung des 4-($C_1$-$C_2$)-alkoxy-3-pyrrolin-2-on-1-yl-essigsäure-(C1-$C_4$)-alkylesters bei Temperaturen von 80 bis 120°C unter vermindertem Druck durchgeführt wird.

5. Verfahren nach Patentansprüchen 2, 3 und 4, dadurch gekennzeichnet, dass die Alkylierung des 4-Benzyloxy-3-pyrrolin-2-ons in polaren aprotischen Lösungsmitteln bei 0 bis 40°C durchgeführt wird.

6. Verfahren nach Patentansprüchen 2, 3, 4 und 5, dadurch gekennzeichnet, daß die Umsetzung des 4-Benzyloxy-3-pyrrolin-2-on-1-yl-essigsäure-($C_1$-$C_4$)-alkyl- oder -benzylesters ohne dessen Isolierung mit Ammoniak, z.B. bei Temperaturen von 60 bis 80°C im Autoklaven erfolgt.

7. Verwendung der Verbindung nach Patentanspruch 1 zur Herstellung von 4-Hydroxy-pyrrolidin-2-on-1-yl-acetamid, dadurch gekennzeichnet, dass das 4-Benzyloxy-3-pyrrolin-2-on-1-yl-acetamid durch katalytische Hydrogenolyse zum intermediären 2,4-Dioxo-pyrrolidin-1-yl-acetamid und weiter entweder durch katalytische Hydrierung oder durch Reduktion mit einem komplexen Borhydrid zum Endprodukt umgesetzt wird.

8. Verwendung nach Patentanspruch 7, dadurch gekennzeichnet, dass mit einem Palladiumkatalysator in polaren aprotischen Lösungsmitteln bei 1 bis 20 bar und bei 0 bis 30°C hydrogenolysiert wird und darauf mit Natriumborhydrid zu Endprodukt reduziert wird.

9. Verwendung nach Patentanspruch 7 , dadurch gekennzeichnet, dass mit einem Palladiumkatalysator in polaren protischen Lösungsmitteln, bei 1 bis 20 bar und bei 0 bis 30°C hydrogenolysiert wird und darauf mit einem Platinkatalysator mit Wasserstoff bei 5 bis 20 bar und Temperaturen zwischen 0 und 30°C zum Endprodukt hydriert wird.

10. Verwendung nach Patentanspruch 7, dadurch gekennzeichnet, dass die katalytische Hydrogenolyse und die katalytische Hydrierung in einem Schritt zum Endprodukt, in Gegenwart eines Palladium/Platinmischkatalysators in polaren protischen Lösungsmitteln bei 5 bis 20 bar und Temperaturen zwischen 0 und 30°C, durchgeführt wird.

**Patentansprüche für den Vertragsstaat: ES**

1. Verfahren zur Herstellung von 4-Benzyloxy-3-pyrrolin-2-on-1-yl-acetamid der Formel

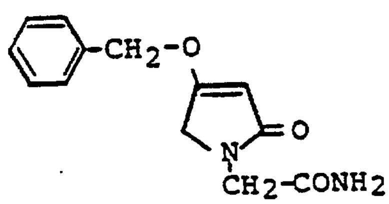

dadurch gekennzeichnet, dass man entweder ein 4-($C_1$-$C_2$)-Alkoxy-3-pyrrolin-2-on mit Benzylalkohol in Gegenwart einer Säure zum 4-Benzyloxy-3-pyrrolin-2-on umestert, mit 2-Bromessigsäure-($C_1$-$C_4$)-alkylester in Gegenwart eines Alkalihydrids zum 4-Benzyloxy-3-pyrrolin-2-on-1-yl-essigsäure-($C_1$-$C_4$)-alkylester alkyliert und schliesslich mit Ammoniak zum Endprodukt umsetzt, oder einen 4-($C_1$-$C_2$)-Alkoxy-3-pyrrolin-2-on-1-yl-essigsäure-($C_1$-$C_4$)-alkylester mit Benzylalkohol in Gegenwart einer Säure zum 4-Benzyloxy-3-pyrrolin-2-on-1-yl-essigsäurebenzylester und weiter – gegebenenfalls ohne dessen Isolierung – mit Ammoniak zum Endprodukt umsetzt.

2. Verfahren nach Patentanspruch 1, dadurch gekennzeichnet, dass als Säure für die Umesterung des 4-($C_1$-$C_4$)-alkoxy-3-pyrrolin-2-ons oder des 4-($C_1$-$C_2$)-alkoxy-3-pyrrolin-2-on-1-yl-essigsäure-($C_1$-$C_4$)-alkylesters Sulfonsäuren eingesetzt werden.

3. Verfahren nach Patentansprüchen 1 und 2, dadurch gekennzeichnet, dass die Umesterung des 4-($C_1$-$C_2$)-alkoxy-3-pyrrolin-2-ons bei Temperaturen von 60 bis 100°C bei vermindertem Druck bzw. die Umesterung des 4-($C_1$-$C_2$)-alkoxy-3-pyrrolin-2-on-1-yl-essigsäure-($C_1$-$C_4$)-alkylesters bei Temperaturen von 80 bis 120°C unter vermindertem Druck durchgeführt wird.

4. Verfahren nach Patentansprüchen 1, 2 und 3 dadurch gekennzeichnet, dass die Alkylierung des 4-Benzyloxy-3-pyrrolin-2-ons in polaren aprotischen Lösungsmitteln bei 0 bis 40°C durchgeführt wird.

5. Verfahren nach Patentansprüchen 1, 2, 3 und 4, dadurch gekennzeichnet, daß die Umsetzung des 4-Benzyloxy-3-pyrrolin-2-on-1-yl-essigsäure-($C_1$-$C_4$)-alkyl oder -benzylesters ohne dessen Isolierung mit Ammoniak, z.B. bei Temperaturen von 60 bis 80°C im Autoklaven erfolgt.

6. Verfahren zur Herstellung von 4-Hydroxy-pyrrolidin-2-on-1-yl-acetamid, dadurch gekennzeichnet, dass 4-Benzyloxy-3-pyrrolin-2-on-1-yl-acetamid durch katalytische Hydrogenolyse zum intermediären 2,4-Dioxo-pyrrolidin-1-yl-acetamid und weiter entweder durch katalytische Hydrierung oder durch Reduktion mit einem komplexen Borhydrid zum Endprodukt umgesetzt wird.

7. Verfahren nach Patentanspruch 6, dadurch gekennzeichnet, dass mit einem Palladiumkatalysator in polaren aprotischen Lösungsmitteln bei 1 bis 20 bar und bei 0 bis 30°C hydrogenolysiert wird und darauf mit, Natriumborhydrid zu Endprodukt reduziert wird.

8. Verfahren nach Patentanspruch 6, dadurch gekennzeichnet, dass mit einem Palladiumkatalysator in polaren protischen Lösungsmitteln, bei 1 bis 20 bar und bei 0 bis 30°C hydrogenolysiert wird und darauf mit einem Platinkatalysator mit Wasserstoff bei 5 bis 20 bar und Temperaturen zwischen 0 und 30°C zum Endprodukt hydriert wird.

9. Verfahren nach Patentanspruch 6, dadurch gekennzeichnet, dass die katalytische Hydrogenolyse und die katalytische Hydrierung in einem Schritt zum Endprodukt, in Gegenwart eines Palladium/Platinmischkatalysators in polaren protischen Lösungsmitteln bei 5 bis 20 bar und Temperaturen zwischen 0 und 30°C, durchgeführt wird.

**Claims for the Contracting States: AT, BE, CH, DE, FR, GB, IT, LI, LU, NL, SE**

1. 4-Benzyloxy-3-pyrrolin-2-one-1-yl-acetamide of the formula

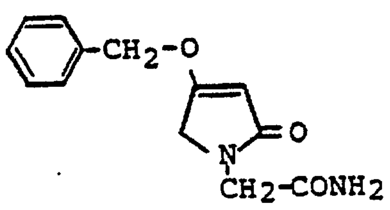

2. Process for the preparation of the compound according to patent claim 1, characterized in that either a 4-($C_1$–$C_2$)-alkoxy-3-pyrrolin-2-one is transesterified with benzyl alcohol in the presence of an acid to prepare 4-benzyloxy-3-pyrrolin-2-one, alkylated with 2-bromoacetic acid-($C_1$–$C_4$)-alkyl ester in the presence of an alkali hydride to prepare the 4-benzyloxy-3-pyrrolin-2-one-1-yl-acetic acid-($C_1$–$C_4$)-alkyl ester and finally reacted with ammonia to the end product or a 4-($C_1$–$C_2$)-alkoxy-3-pyrrolin-2-one-1-yl-acetic acid-($C_1$–$C_4$)-alkyl ester is reacted with benzyl alcohol in the presence of an acid to prepare 4-benzyloxy-3-pyrrolin-2-one-1-yl-acetic acid benzyl ester followed – if desired without isolation thereof – by further reaction with ammonia to obtain the end product.

3. Process according to patent claim 2, characterized in that for the transesterification of the 4-($C_1$–$C_2$)-alkoxy-3-pyrrolin-2-one or the 4-($C_1$–$C_2$)-alkoxy-3-pyrrolin-2-one-1-yl-acetic acid-($C_1$–$C_4$)-alkyl ester sulfonic acids are used as acid.

4. Process according to patent claims 2 and 3, characterized in that the transesterification of the 4-($C_1$–$C_2$)-alkoxy-3-pyrrolin-2-one is carried out at temperatures of 60 to 100°C at reduced pressure and that the transesterification of the 4-($C_1$–$C_2$)-alkoxy-3-pyrrolin-2-one-1-yl-acetic acid-($C_1$–$C_4$)-alkyl ester is carried out at temperatures of 80 to 120°C at reduced pressure resp.

5. Process according to patent claims 2, 3 and 4, characterized in that the alkylation of the 4-benzyloxy-3-pyrrolin-2-one is carried out in a polar aprotic solvent at 0 to 40°C.

6. Process according to patent claims 2,3,4 and 5, characterized in that the reaction of the 4-benzyloxy-3-pyrrolin-2-one-1-yl-acetic acid-($C_1$–$C_4$)-alkyl- or -benzyl ester is effected without isolation thereof with ammonia, e.g. at temperatures of 60 to 80°C in an autoclave.

7. The use of the compound according to patent claim 1 for the preparation of 4-hydroxy-pyrrolidin-2-one-1-yl-acetamide, characterized in that the 4-benzyloxy-3-pyrrolin-2-one-1-yl-acetamide is reacted by catalytic hydrogenolysis to the intermediate 2, 4-dioxo-pyrrolidin-1-yl-acetamide and further reacted either by catalytic hydrogenation or by reduction with a complex borohydride to obtain the end product.

8. The use according to patent claim 7, characterized in that the hydrogenolysis is effected with a

palladium catalyst in polar aprotic solvents at 1 to 20 bar and at 0 to 30°C, followed by the reduction with sodium borohydride to obtain the end product.

9. The use according to patent claim 7, characterized in that the hydrogenolysis is effected with a palladium catalyst in polar protic solvents at 1 to 20 bar and at 0 to 30°C whereupon the hydrogenation is effected with a platinum catalyst with hydrogen at 5 to 20 bar and at temperatures between 0 and 30°C to obtain the end product.

10. The use according to patent claim 7, characterized in that the catalytic hydrogenolysis and the catalytic hydrogenation are effected in one step in the presence of a mixed palladium/ platinum catalyst in polar protic solvents at 5 to 20 bar and at temperatures between 0 and 30°C.

**Claims for the contracting State: ES**

1. Process for the preparation of 4-benzyloxy-3-pyrrolin-2-one1-yl-acetamide of the formula:

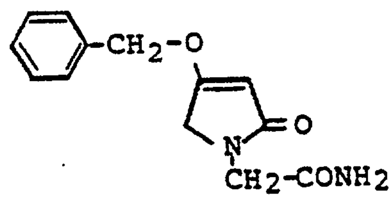

characterized in that either a 4-($C_1$–$C_2$)-alkoxy-3-pyrrolin-2-one is transesterified with benzyl alcohol in the presence of an acid to prepare 4-benzyloxy-3-pyrrolin-2-one, alkylated with 2-bromoacetic acid-($C_1$–$C_4$)-alkyl ester in the presence of an alkali hydride to prepare the 4-benzyloxy-3-pyrrolin-2-one-1-yl-acetic acid-($C_1$–$C_4$)-alkyl ester and finally reacted with ammonia to the end product or a 4-($C_1$–$C_2$)-alkoxy-3-pyrrolin-2-one-1-yl-acetic acid-($C_1$–$C_4$)-alkyl ester is reacted with benzyl alcohol in the presence of an acid to prepare 4-benzyloxy-3-pyrrolin-2-one-1-yl-acetic acid benzyl ester followed – if desired without isolation thereof by further reaction with ammonia to obtain the end product.

2. Process according to patent claim 1, characterized in that for the transesterification of the 4-($C_1$–$C_2$)-alkoxy-3-pyrrolin-2-one or the 4-($C_1$–$C_2$)-alkoxy-3-pyrrolin-2-one-1-yl-acetic acid-($C_1$–$C_4$)-alkyl ester sulfonic acids are used as acid.

3. Process according to patent claims 1 and 2, characterized in that the transesterification of the 4-($C_1$–$C_2$)-alkoxy-3-pyrrolin-2-one is carried out at temperatures of 60 to 100°C at reduced pressure and that the transesterification of the 4-($C_1$–$C_2$-)alkoxy-3-pyrrolin-2-one-1-yl-acetic acid-($C_1$–$C_4$)-alkyl ester is carried out at temperatures of 80 to 120°C at reduced pressure resp.

4. Process according to patent claims 1,2 and 3, characterized in that the alkylation of the 4-benzyloxy-3-pyrrolin-2-one is carried out in a polar aprotic solvent at 0 to 40°C.

5. Process according to patent claims 1,2,3 and 4, characterized in that the reaction of the 4-benzyloxy-3-pyrrolin-2-one-1-yl-acetic acid-($C_1$–$C_4$)-alkyl- or -benzyl ester is effected without isolation thereof with ammonia, e.g. at temperatures of 60 to 80°C in an autoclave.

6. Process for the preparation of 4-hydroxy-pyrrolidin-2-one-1-yl-acetamide, characterized in that 4-benzyloxy-3-pyrrolin-2-one-l-yl-acetamide is reacted by catalytic hydrogenolysis to obtain the intermediate 2, 4-dioxo-pyrrolidin-1-yl-acetamide and then further reacted by catalytic hydrogenation or by reduction with a complex borohydride to obtain the end product.

7. Process according to patent claim 6, characterized in that the hydrogenolysis is effected with a palladium catalyst in a polar aprotic solvent at 1 to 20 bar and at 0 to 30°C whereupon the reduction with sodium borohydride is carried out to obtain the end product.

8. Process according to patent claim 6, characterized in that the hydrogenolysis is effected with a palladium catalyst in a polar protic solvent at 1 to 20 bar and at 0 to 30°C whereupon the hydrogenation is carried out with a platinum catalyst with hydrogen at 5 to 20 bar and at temperatures between 0 and 30°C to obtain the end product.

9. Process according to patent claim 6, characterized that the catalytic hydrogenolysis and the catalytic hydrogenation to obtain the end product are carried out in one step in the presence of a mixed palladium/platinum catalyst in polar protic solvents at 5 to 20 bar and at temperatures between 0 and 30°C.

**Revendications pour les Etats contractants: AT, BE, CH, DE, FR, GB, IT, LI, LU, NL, SE**

1. 4-benzyloxy-3-pyrroline-one-1-yl-acétamide de formule

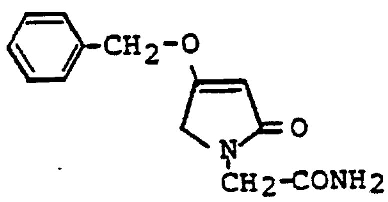

2. Procédé de préparation du composé selon la revendication 1, caractérisé en ce que l'on transestérifie une 4-($C_1$–$C_2$)-alcoxy-3-pyrroline-2-one avec l'alcool benzylique en présence d'un acide en la 4-benzyloxy-3-pyrroline-2-one, on l'alkyle avec un ($C_1$–$C_4$)-alkylester de l'acide 2-bromacétique en présence d'un hydrure alcalin en un ($C_1$–$C_4$)-alkylester de l'acide 4-benzyloxy-3-pyrroline-2-one-1-yl-acétique et enfin on la transforme en produit final avec l'ammoniac, ou bien on transforme un ($C_1$–$C_4$)-alkylester de l'acide 4-($C_1$–$C_2$)-alcoxy-3-pyrroline-2-one-1-yl-acétique avec l'alcool benzylique en présence d'un acide en le benzylester de l'acide 4-benzyloxy-3-pyrroline-2-one-1-yl-acétique et ensuite – éventuellement sans l'isoler - avec l'ammoniac en le produit final.

3. Procédé selon la revendication 2, caractérisé en ce que comme acide pour la transestérification

de la 4-($C_1$–$C_2$)-alcoxy-3-pyrroline-2-one ou du ($C_1$–$C_4$)-alkylester de l'acide 4-($C_1$–$C_2$)-alcoxy-3-pyrroline-2-one-1-yl-acétique on utilise des acides sulfoniques.

4. Procédé selon les revendications 2 et 3, caractérisé en ce que la transestérification de la 4-($C_1$–$C_2$)-alcoxy-3-pyrroline-2-one a lieu à des températures de 60 à 100°C sous pression réduite et en ce que la transestérification du ($C_1$–$C_4$)-alkylester de l'acide 4-($C_1$–$C_2$)-alcoxy-3-pyrroline-2-one-1-yl-acétique a lieu à des températures de 80 à 120°C sous pression réduite.

5. Procédé selon les revendications 2, 3 et 4, caractérisé en ce que l'alkylation de la 4-benzyloxy-3-pyrroline-2-one a lieu dans des solvants polaires aprotiques entre 0 et 40°C.

6. Procédé selon les revendications 2, 3, 4 et 5, caractérisé en ce que la conversion du ($C_1$–$C_4$)-alkyl- ou benzylester de l'acide 4-benzyloxy-3-pyrroline-2-one-1-yl-acétique a lieu avec l'ammoniac sans son isolement, par exemple à des températures de 60 à 80°C en autoclave.

7. Utilisation du composé selon la revendication 1 pour la préparation du 4-hydroxy-pyrrolidine-2-one-1-yl-acétamide, caractérisée en ce que l'on transforme le 4-benzyloxy-3-pyrroline-2-one-1-yl-acétamide par hydrogénolyse catalytique en le 2, 4-dioxo-pyrrolidine-1-yl-acétamide intermédiaire et ensuite en le produit final par hydrogénation catalytique ou par réduction avec un borohydrure complexe.

8. Utilisation selon la revendication 7, caractérisée en ce que l'on effectue l'hydrogénolyse avec un catalyseur au palladium dans des solvants polaires aprotiques entre 1 et 20 bars et entre 0 et 30°C puis l'on réduit en le produit final avec le borohydrure de sodium.

9. Utilisation selon la revendication 7, caractérisée en ce que l'on effectue l'hydrogénolyse avec un catalyseur au palladium dans des solvants polaires protiques entre 1 et 20 bars et entre 0 et 30°C puis l'on hydrogène en le produit final avec un catalyseur au platine et avec de l'hydrogène entre 5 et 20 bars et à des températures comprises entre 0 et 30°C.

10. Utilisation selon la revendication 7, caractérisée en ce que l'on effectue l'hydrogénolyse catalytique et l'hydrogénation catalytique en le produit final en une étape, en présence d'un catalyseur mixte palladium/platine entre 5 et 20 bars et à des températures comprises entre 0 et 30°C.

**Revendications pour l'Etat contractant: ES**

1. Procédé de préparation du 4-benzyloxy-3-pyrroline-2-one-1-yl-acétamide de formule

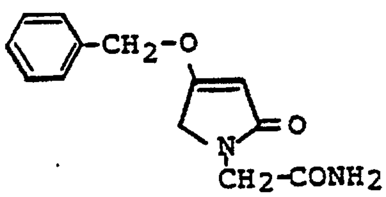

caractérisé en ce que l'on transestérifie une 4-($C_1$–$C_2$)-alcoxy-3-pyrroline-2-one avec l'alcool benzylique en présence d'un acide en la 4-benzyloxy-3-pyrroline-2-one, on l'alkyle avec un ($C_1$–$C_4$)-alkylester de l'acide 2-bromo-acétique en présence d'un hydrure alcalin en un ($C_1$–$C_4$)-alkylester de l'acide 4-benzyloxy-3-pyrroline-2-one-1-yl-acétique et enfin on la transforme en produit final avec l'ammoniac, ou bien on transforme un ($C_1$–$C_4$)-alkylester de l'acide 4-($C_1$–$C_2$)-alcoxy-3-pyrroline-2-one-1-yl-acétique avec l'alcool benzylique en présence d'un acide en le benzylester de l'acide 4-benzyloxy-3-pyrroline-2-one-1-yl-acétique et ensuite – éventuellement sans l'isoler – avec l'ammoniac en le produit final.

2. Procédé selon la revendication 1, caractérisé en ce que comme acide pour la transestérification de la 4-($C_1$–$C_2$)-alcoxy-3-pyrroline-2-one ou du ($C_1$–$C_4$)-alkylester de l'acide 4-($C_1$–$C_2$)-alcoxy-3-pyrroline-2-one-1-yl-acétique on utilise des acides sulfoniques.

3. Procédé selon les revendications 1 et 2, caractérisé en ce que la transestérification de la 4-($C_1$–$C_2$)-alcoxy-3-pyrroline-2-one a lieu à des températures de 60 à 100°C sous pression réduite et en ce que la transestérification du ($C_1$–$C_4$)-alkylester de l'acide 4-($C_1$–$C_2$)-alcoxy-3-pyrroline-2-one-1-yl-acétique a lieu à des températures de 80 à 120°C sous pression réduite.

4. Procédé selon les revendications 1, 2 et 3, caractérisé en ce que l'alkylation de la 4-benzyloxy-3-pyrroline-2-one a lieu dans des solvants polaires aprotiques entre 0 et 40°C.

5. Procédé selon les revendications 1, 2, 3 et 4, caractérisé en ce que la conversion du ($C_1$–$C_4$)-alkyl- ou benzylester de l'acide 4-benzyloxy-3-pyrroline-2-one-1-yl-acétique a lieu avec l'ammoniac sans son isolement, par exemple à des températures de 60 à 80°C en autoclave.

6. Procédé de préparation du 4-hydroxy-pyrrolidine-2-one-1-yl-acétamide, caractérisé en ce que l'on transforme le 4-benzyloxy-3-pyrroline-2-one-1-yl-acétamide par hydrogénolyse catalytique en le 2,4-dioxo-pyrrolidine-1-yl-acétamide intermédiaire et ensuite en le produit final par hydrogénation catalytique ou par réduction avec un borohydrure complexe.

7. Procédé selon la revendication 6, caractérisé en ce que l'on effectue l'hydrogénolyse avec un catalyseur au palladium dans des solvants polaires aprotiques entre 1 et 20 bars et entre 0 et 30°C puis

l'on réduit en le produit final avec le borohydrure de sodium.

8. Procédé selon la revendication 6, caractérisé en ce que l'on effectue l'hydrogénolyse avec un catalyseur au palladium dans des solvants polaires protiques entre 1 et 20 bars et entre 0 et 30°C puis l'on hydrogène en le produit final avec un catalyseur au platine et avec de l'hydrogène entre 5 et 20 bars et à des températures comprises entre 0 et 30°C.

9. Procédé selon la revendication 6, caractérisé en ce que l'on effectue l'hydrogénolyse catalytique et l'hydrogénation catalytique en le produit final en une étape, en présence d'un catalyseur mixte palladium/platine dans des solvants polaires protiques entre 5 et 20 bars et à des températures comprises entre 0 et 30°C.